# EUROPEAN PATENT APPLICATION

(11) **EP 1 167 338 A1**
(43) Date of publication of application: **02.01.2002**
(21) Application number: 00305404.6
(22) Date of filing: 27.06.2000
(51) Int. Cl.: C07C 67/31, C07C 69/708, C07C 67/03, C07C 231/02, C10M 105/34, A61K 7/00, A23L 1/00

(54) **Fatty acid alternative**

(71) Applicant: Fina OleoChemicals N.V., 9940 Ertvelde (BE)
(72) Inventor: Packet, Dirk, 1703 Asse (BE)
(74) Representative: Daniels, Jeffrey Nicholas

(57) **Abstract**

Provided is a process for the production of an ester of an etheracid, which process comprises reacting:
(a) a compound comprising an olefin group and/or an alcohol group, and having from 2-24 carbon atoms with
(b) a hydroxy acid ester compound having from 2-8 carbon atoms, excluding the carbon atoms of the ester moiety;
wherein the reaction is carried out in the presence of a heterogeneous acid catalyst, or a homogeneous superacid catalyst, and wherein the compounds (a) and (b) may independently be linear or branched, and/or substituted or unsubstituted.

## Description

The present invention provides a process for the production of an ester of an ether acid, which ester of an ether acid is useful as a low cost replacement for natural fatty acids and their derivatives. The invention also provides a process for transesterification and production of a primary amide using the etheracid ester product.

In oleochemistry, the ideal fatty acid is a saturated acid with good cold stability (low melting point) and good oxidation stability. In practice, the only natural fatty acids that satisfy these two criteria are those present in the C₈-C₁₀ fraction found, for example, in coconut oil and palm kernel oil. The properties of some of these acids are shown in Table 1.

**Table 1**

| | **Melting point/°C** | **Content in coconut oil/%** | **Content in palm kernel oil/%** |
|---|---|---|---|
| C8 (caprylic acid) | 16.0 | 8 | 3 |
| C10 (capric acid) | 31.6 | 6 | 3 |
| C12 (lauric acid) | 44.2 | | |

As can be seen from this data, lauric acid is already solid at ambient temperature. Due to their good oxidation and cold stability, the C₈-C₁₀ fatty acid fraction is widely used in the production of fatty acid derivatives used as lubricants, in cosmetic compositions, as food additives or in other industrial applications where these properties are desirable. However, this C₈-C₁₀ fraction is rather scarce in nature, as can be seen from the above Table. As a consequence there is a shortage of the C₈-C₁₀ fraction (either as fatty acid or as methyl ester, coming from the fatty alcohol plants that use coconut oil or palm kernel oil as feed), resulting in high market prices.

It is possible to produce alternatives to these fatty acids by synthetic means, but up to present these methods have been expensive, or environmentally unsound. A well known method for producing ethers is the alkoxymercuration/demercuration reaction. This reaction involves reacting an alkene with an alcohol in the presence of a mercury-containing catalyst, such as mercuric trifluoroacetate. The reaction is a two stage process in which firstly the alcohol and mercury species react with the alkene to bond with the alkene carbon atoms. Subsequently, a demercuration step is carried out using, for example sodium borohydride. However this reaction produces elemental mercury, which is unacceptable on an industrial scale. It is hazardous to the environment, expensive to remove and presents clear problems if the product is to be used, for example, as a food additive.

It is an object of the present invention to solve the problems associated with the above prior art. Thus, it is an object of the present invention to provide a cheaper alternative to natural fatty acids, by providing a synthetic route that is cheaper and more environmentally acceptable that known synthetic routes.

Accordingly, the present invention provides a process for the production of an ester of an etheracid, which process comprises reacting:
(a) a compound comprising an olefin group and/or an alcohol group, and having from 2-24 carbon atoms with
(b) a hydroxy acid ester compound having from 2-8 carbon atoms, excluding the carbon atoms of the ester moiety;
wherein the reaction is carried out in the presence of a heterogeneous acid catalyst, or a homogeneous superacid catalyst, and wherein the compounds (a) and (b) may independently be linear or branched, and/or substituted or unsubstituted.

Thus, this invention provides a process for the production of esters of ether acids, that have properties which are very close to those of C₈-C₁₀ esters, and in particular their methyl esters. The process has the further advantage of being relatively inexpensive and straightforward, in contrast to known synthetic routes. These esters of etheracids can be produced at a price below the market price of C₈-C₁₀ fatty acids, or their esters.

The compounds (a) and (b) may be linear or branched or substituted or unsubstituted, as discussed above. The substituents are not particularly limited, provided that the final products have the desired properties, and may comprise any organic group and/or one or more atoms from any of groups IIIA, IVA, VA, VIA or VIIA of the Periodic Table, such as a B, Si, N, P, O, or S atom or a halogen atom (e.g. F, C1, Br or I).

When the substituent comprises an organic group, the organic group preferably comprises a hydrocarbon group. The hydrocarbon group may comprise a straight chain, a branched chain or a cyclic group. Independently, the hydrocarbon group may comprise an aliphatic or an aromatic group. Also independently, the hydrocarbon group may comprise a saturated or unsaturated group.

When the hydrocarbon comprises an unsaturated group, it may comprise one or more alkene functionalities and/or one or more alkyne functionalities. When the hydrocarbon comprises a straight or branched chain group, it may comprise one or more primary, secondary and/or tertiary alkyl groups. When the hydrocarbon comprises a cyclic group it may comprise an aromatic ring, an aliphatic ring, a heterocyclic group, and/or fused ring derivatives of these groups. The cyclic group may thus comprise a benzene, naphthalene, anthracene, indene, fluorene, pyridine, quinoline, thiophene, benzothiophene, furan, benzofuran, pyrrole, indole, imidazole, thiazole, and/or an oxazole group, as well as regioisomers of the above groups.

The number of carbon atoms in the hydrocarbon group is not especially limited, but should comprise from 1-22 C atoms. The hydrocarbon group may thus be a lower hydrocarbon (1-6 C atoms) or a higher hydrocarbon (7-22 C atoms). The number of atoms in the ring of the cyclic group is not especially limited, but preferably the ring of the cyclic group comprises from 3-10 atoms, such as 3, 4, 5, 6 or 7 atoms.

The groups comprising heteroatoms described above, as well as any of the other groups defined above, may comprise one or more heteroatoms from any of groups IIIA, IVA, VA, VIA or VIIA of the Periodic Table, such as a B, Si, N, P, O, or S atom or a halogen atom (e.g. F, C1, Br or I). Thus the substituent may comprise one or more of any of the common functional groups in organic chemistry, such as hydroxy groups, carboxylic acid groups, ester groups, ether groups, aldehyde groups, ketone groups, amine groups, amide groups, imine groups, thiol groups, thioether groups, sulphate groups, sulphonic acid groups, and phosphate groups etc. The substituent may also comprise derivatives of these groups, such as carboxylic acid anhydrydes and carboxylic acid halides. It is especially preferred, though, that the substituent comprises a hydrocarbon group.

In addition, any substituent may comprise a combination of two or more of the substituents and/or functional groups defined above.

In a preferred embodiment of the present invention, the process comprises reacting:
(a) a compound of formula (I): 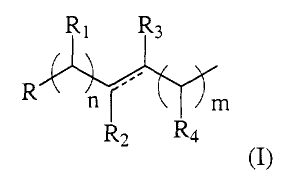 in which n is an integer of 0-22; m is an integer of 0-22 provided that n+m is 22 or less; the carbon atoms joined by the dotted line may be joined by a double bond or a single bond; and when these carbon atoms are joined by a double bond R is H and when these carbon atoms are joined by a single bond, R is OH; and R¹, R², R³ and R⁴ are independently selected from H, halogen, and a substituent comprising a carbon atom, such as a substituent as defined above;
   with
(b) a compound of formula (II): 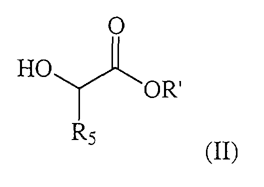 in which R' is an alkyl group having from 1-8 carbon atoms; and R⁵ comprises H, halogen, or a substituent comprising a carbon atom, such as a substituent defined above

It is particularly preferred that the R' group is an alkyl group having from 1-8 carbon atoms. Most preferably R' is a methyl group, but R' may also be an ethyl, n-propyl, iso-propyl, n-butyl, iso-butyl or t-butyl group, if desired.

In preferred embodiments of the invention, the compounds (a) and (b) are unsubstituted (wherein R¹, R², R³, R⁴ and R⁵ are each H) and also linear. Particularly preferred as compound (b) is methylglycolate. Particularly preferred as compound (a) are C₂-C₁₀ α-olefins, such as ethene, propene, 1-butene, 1-pentene, 1-hexene, 1-pentene, 1-octene, 1-nonene, and 1-decene.

When the products of the present process are intended to replace C₈-C₁₀ product fractions, the product ester of an etheracid is preferably a compound comprising from 8-12 carbon atoms, excluding the carbon atoms of the ester moiety. However, the final product may have from 4-26 carbon atoms, if desired, excluding the carbon atoms of the ester moiety.

The catalyst used in the present process is a heterogeneous acid catalyst or may be a homogeneous superacid catalyst. Preferably the catalyst comprises a zeolite, an acid resin or a solid amorphous catalyst, such as silica-alumina. When the catalyst comprises a zeolite, preferably the zeolite comprises a Y-type zeolite. Superacids (solutions of strong acids in very acidic solvents) such as SbF₅ in HF (SbF₆⁻ + H₂F⁺) and SbF₅ in HSO₃F (FSO₃SbF₅⁻ + H₂SO₃F⁺) or other superacids, may also be employed as catalysts, if desired.

The temperature of the present process is not especially limited, provided that the process proceeds to form the desired etheracid ester. However, preferably the compounds (a) and (b) are reacted at a temperature of from 100-240°C. More preferably the temperature employed is from 120-200°C.

The present process may be extended by adding further steps, to produced further esters, or amide compounds, such as primary amides. Thus the present process may further comprise performing a transesterification step on the product etheracid ester, to produce a further ester of an etheracid. The present process may also further comprise a step of forming a primary amide compound from the product etheracid ester. Preferably, the primary amide compound is a low melting point primary amide compound. Such compounds are useful as lubricants.

As discussed above, the present etheracid ester compounds, and their derivatives, have a wide variety of uses. In particular they may be used as a replacement for a natural fatty acid or its derivative in a composition, or to improve the cold stability of a composition. Typically these compounds are useful as lubricants, in cosmetics compositions, as food additives, or in compositions requiring good cold stability.

The present invention will be further described by way of example only, with reference to the following specific embodiments.

### Examples

### Example 1

1 mole of methylglycolate was reacted with 2 moles of 1-octene (1 mole excess) using 1 % zeolite BB6 from PQ corporation (BB6 is a Y-type zeolite) as catalyst. The reaction was carried out at 180°C for 3 hours. the reaction product was found to contain 67.8 % of methyl esters of etheracids, as determined by GLC. These products were separated from unreacted methylglycolate by simple fractionation. The methyl ester of this etheracid with 10 carbon atoms has a solidification point of -39°C, whilst the melting point of its natural equivalent methyldecanoate is only -18°C

### Example 2

The same reaction was carried out as performed in Example 1, except a temperature of 130°C was employed and Amberlyst 15 from Rohm & Haas was employed as catalyst (an acid resin). The reaction product was found to comprise 15.6 % of methyl esters of etheracids as determined by GLC.

Both of these reactions demonstrate that large quantities of esters of etheracids can be produced using widely available starting materials and catalysts, in a simple cost effective reaction. Even at temperatures as low as 130°C, a higher percentage of product can be formed than is present in natural products.

## Claims

1. A process for the production of an ester of an etheracid, which process comprises reacting:
(a) a compound comprising an olefin group and/or an alcohol group, and having from 2-24 carbon atoms with
(b) a hydroxyacid ester compound having from 2-8 carbon atoms, excluding the carbon atoms of the ester moiety;
wherein the reaction is carried out in the presence of a heterogeneous acid catalyst, or a homogeneous superacid catalyst, and wherein the compounds (a) and (b) may independently be linear or branched, and/or substituted or unsubstituted.

2. A process according to claim 1, which process comprises reacting:
(a) a compound of formula (I): 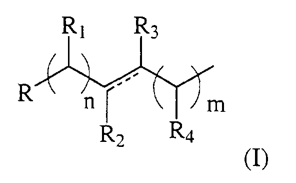 wherein n is an integer of 0-22; m is an integer of 0-22; n+m is 22 or less; the carbon atoms joined by the dotted line may be joined by a double bond or a single bond; when these carbon atoms are joined by a double bond R comprises H; when these carbon atoms are joined by a single bond, R comprises OH; and R¹, R², R³ and R⁴ are independently selected from H, halogen, and a substituent comprising a hydrocarbon group; with
(b) a compound of formula (II): 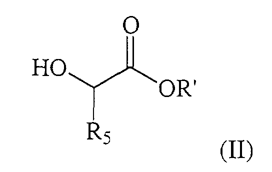 in which R' is an alkyl group having from 1-8 carbon atoms; and R⁵ comprises H, halogen, or a substituent comprising a hydrocarbon group.

3. A process according to claim 1, wherein R' is a methyl group.

4. A process according to any preceding claim, wherein R¹, R², R³, R⁴ and R⁵ are each H.

5. A process according to any preceding claim, wherein the product ester of an etheracid is a compound comprising from 8-12 carbon atoms, excluding the carbon atoms of the ester moiety.

6. A process according to any preceding claim, wherein the heterogeneous acid catalyst comprises a zeolite, an acid resin or a solid amorphous catalyst, such as silica-alumina.

7. A process according to claim 6, wherein the zeolite comprises a Y-type zeolite.

8. A process according to any preceding claim, wherein the compounds (a) and (b) are reacted at a temperature of from 100-240°C.

9. A process according to any preceding claim, which process further comprises performing a transesterification step on the product etheracid ester, to produce a further ester of an ether acid.

10. A process according to any of claims 1-8, which process further comprises forming a primary amide compound from the product etheracid ester.

11. A process according to claim 10, wherein the primary amide compound is a low melting point primary amide compound.

12. Use of an ester of an ether acid, or a primary amide of an ether acid, obtainable according to a process as defined in any preceding claim, as a replacement for a natural fatty acid or its derivative.

13. Use of an ester of an ether acid, or a primary amide of an ether acid, obtainable according to a process as defined in any of claims 1-11, to improve the cold stability of a composition.

14. Use of an ester of an ether acid, or a primary amide of an ether acid, obtainable according to a process as defined in any of claims 1-11, as a lubricant, in a cosmetics composition, as a food additive, or in a composition requiring good cold stability.

15. A lubricant composition comprising an ester of an ether acid, obtainable according to a process as defined in any of claims 1-11.

16. A cosmetics composition comprising an ester of an ether acid, obtainable according to a process as defined in any of claims 1-11.

17. A food additive composition comprising an ester of an ether acid, obtainable according to a process as defined in any of claims 1-11.
